# EUROPÄISCHE PATENTANMELDUNG

(11) **EP 3 715 825 A1**
(43) Veröffentlichungstag der Anmeldung: **30.09.2020**
(21) Anmeldenummer: 20163682.6
(22) Anmeldetag: 17.03.2020
(51) Int. Cl.: G01N 11/16, G01N 33/44

(54) **VERFAHREN ZUM BERÜHRUNGSLOSEN BESTIMMEN DER SCHWINDUNG VON HARZ UND VERWENDUNG EINER VORRICHTUNG ZUR DURCHFÜHRUNG DES VERFAHRENS**

(30) Priorität: 28.03.2019 DE 102019107995
(71) Anmelder: Deutsches Zentrum für Luft- und Raumfahrt e.V., 51147 Köln (DE)
(72) Erfinder: Mahrholz, Thorsten, 38228 Salzgitter (DE); von Monkiewitsch, Monika, 38162 Cremlingeng (DE)
(74) Vertreter: REHBERG HÜPPE + PARTNER

(57) **Zusammenfassung**

Zum Bestimmen der Schwindung eines Harzes (10) beim Aushärten (23) wird aus dem Harz (10) ein Tropfens (7) ausgebildet, und ein frühes Erfassen eines Volumens des Tropfens (7) wird durchgeführt. Dann wird das Harz (10) ausgehärtet (23). Hieran schließt sich ein spätes Erfassen des Volumens des Tropfens (7) an. Dabei wird der Tropfen (7) von dem frühen Erfassen (22) bis zu dem späten Erfassen durch Levitation (26) in Schwebe gehalten; und sowohl bei dem frühen Erfassen (22) als auch dem späten Erfassen (24) des Volumens werden die Form und die Abmessungen des Tropfens (7) in allen drei Raumrichtungen erfasst.

## Beschreibung

### TECHNISCHES GEBIET DER ERFINDUNG

Die Erfindung bezieht sich auf ein Verfahren zum Bestimmen der Schwindung eines Harzes, d. h. von Harz einer vorgegebenen Zusammensetzung, beim Aushärten. Dieses Verfahren umfasst die Schritte Ausbilden eines Tropfens aus dem Harz, frühes Erfassen eines Volumens des Tropfens vor dem Aushärten des Harzes, Aushärten des Harzes und spätes Erfassen des Volumens des Tropfens nach dem Aushärten des Harzes. Weiterhin bezieht sich die Erfindung auf die Verwendung einer Vorrichtung bei der Durchführung eines solchen Verfahrens.

### STAND DER TECHNIK

Die Schwindung von Harz während des Aushärtens führt in Faserverbundwerkstoffen, bei denen das Harz eine Verstärkungsfasern einbettende Matrix ausbildet, zu inhärenten werkstofflichen Eigenspannungen. Diese Eigenspannungen können sich einerseits in Faserondulationen, d. h. wellenartigen Verformungen der Verstärkungsfasern und andererseits in einer Vorspannung in der Matrix auswirken. Beide Auswirkungen reduzieren die Maximallasten, für die der Faserverbundwerkstoff geeignet ist. Dies ist gleichbedeutend damit, dass der werkstoffliche Ausnutzungsgrad des Faserverbundwerkstoffs reduziert ist.

Es ist bekannt, durch eine angepasste Temperaturführung, durch eine geschickte Auswahl der das Harz ausbildenden Monomere und durch einen Zuschlag von Füllstoffen die Schwindung des Harzes beim Aushärten zu reduzieren. Um den Erfolg dieser Maßnahmen beurteilen zu können und besser geeignete Maßnahmen entwickeln zu können, fehlt es aber an einem alltagstauglichen Messverfahren zur exakten und vergleichbaren Bestimmung der Schwindung des Harzes.

NAWAB, Yasir, et al.: Chemical shrinkage characterization techniques for thermoset resins and associated composites. In: Journal of Materials Science, Vol. 48, 2013, No. 16, S. 5387-5409, beschreiben verschiedene bekannte Verfahren zum Bestimmen der Schwindung von Harz beim Aushärten. Hiervon haben die Bestimmung der Harzschwindung mittels Auftriebswaage und ein Verfahren unter Verwendung eines Pyknometers Eingang in die Normung gefunden, siehe DIN EN ISO 3521:1999-10 und DIN EN ISO 1675:1998-10. Beide Verfahren sind jedoch wegen der nur diskontinuierlich möglichen Datenerfassung für einen universellen Einsatz nicht geeignet. Andere bekannte Verfahren liefern keine untereinander vergleichbaren Ergebnisse, weil sie insbesondere bei kleinen betrachteten Mengen des Harzes inhärent das Messergebnis beeinflussen, beispielsweise bei Verfahren unter Verwendung eines Kontaktwinkelmessgeräts.

Aus der EP 0 275 825 A1 ist ein Verfahren zur Messung von Volumenänderungen, insbesondere Schwindungsmessungen an Duroplasten während der Härtung, bekannt. Das auszumessende Material wird mit einem solchen Druck beaufschlagt, dass eine völlig homogene Probe ohne Riss- und Blasenbildung erzielt wird. Gleichzeitig und unabhängig voneinander werden unter nicht isothermen Bedingungen der aufgebrachte Druck, der durch die Probe auf den Boden ausgeübte Druck, die Temperatur in der Probe und in deren Umgebung und das Volumen der Probe gemessen und einer Recheneinheit zugeführt. Eine Vorrichtung zur Durchführung dieses Verfahrens weist einen Messzylinder mit einer Messbohrung und einen in die Messbohrung passenden, druckbeaufschlagten Messkolben sowie einen dem Messkolben zugeordneten Wegaufnehmer, eine Temperatursonde in dem Messzylinder, eine Temperatursonde in der Messbohrung zur Messung der Temperatur in der Probe, einen Druckmesser in der Druckluftzufuhrleitung, einen Druckaufnehmer an dem dem Messkolben entgegengesetzten Ende der Messbohrung und eine Heizung für den Messzylinder auf. Mit den die Probe kontaktierenden Teilen der Messvorrichtung nimmt das Verfahren Einfluss auf die Probe. Eine für dieses bekannte Verfahren geeignete Probe weist zudem erhebliche Abmessungen auf.

EXNER, Wibke, et al.: Determination of volumetric shrinkage of thermally cured thermosets: Test method. In: Polymer Testing, Vol. 49, 2016, S. 100-106, beschreiben die Bestimmung der Schwindung eines Harzes beim Aushärten durch Beobachten eines auf einem Probenhalter angeordneten Tropfens des Harzes mit einer Videokamera. Der zylindrische Probenhalter von festem Durchmesser ist mit Referenzmarkierungen versehen, die zusätzlich zu dem Tropfen mit der Videokamera erfasst werden und die eine genaue Bestimmung des Durchmessers und der Höhe des Tropfens auf den Probenhalter ermöglichen.

### AUFGABE DER ERFINDUNG

Der Erfindung liegt die Aufgabe zugrunde, ein Verfahren zum Bestimmen der Schwindung eines Harzes beim Aushärten aufzuzeigen, das für einen universellen Einsatz mit kleinen Mengen des zu untersuchenden Harzes geeignet ist und eine kontinuierliche Messwertaufnahme erlaubt.

### LÖSUNG

Die Aufgabe der Erfindung wird durch ein Verfahren mit den Merkmalen des unabhängigen Patentanspruchs 1 gelöst. Die Patentansprüche 2 bis 10 betreffen bevorzugte Ausführungsformen des erfindungsgemäßen Verfahrens. Patentanspruch 11 ist auf die Verwendung einer Vorrichtung zur Durchführung des erfindungsgemäßen Verfahrens gerichtet. Die Patentansprüche 12 bis 15 betreffen bevorzugte Ausführungsformen der verwendeten Vorrichtung.

### BESCHREIBUNG DER ERFINDUNG

Bei einem erfindungsgemäßen Verfahren zum Bestimmen der Schwindung eines Harzes beim Aushärten mit den Schritten Ausbilden eines Tropfens aus dem Harz, frühes Erfassen eines Volumens des Tropfens vor dem Aushärten des Harzes, Aushärten des Harzes und spätes Erfassung des Volumens des Tropfens nach dem Aushärten des Harzes wird der Tropfen zumindest von dem frühen Erfassen bis zu dem späten Erfassen durch Levitation in Schwebe gehalten und werden bei dem frühen Erfassen und dem späten Erfassen des Volumens Form und Abmessungen des Tropfens in allen drei Raumrichtungen erfasst. Dabei bedeutet, dass der Tropfen von dem frühen Erfassen bis zu dem späten Erfassen durch Levitation in Schwebe gehalten wird, dass er auch während des frühen und des späten Erfassens levitiert wird.

### WEITERER STAND DER TECHNIK

Aus R. Tuckermann et al.: "Levitation in Ultraschallfeldern: Schwebende Tröpfen", Physik in unserer Zeit 32.2 (2001), 69-75 ist es bekannt, dass man in starken stehenden Ultraschallfeldern feste, flüssige und gasförmige Proben berührungslos schweben lassen kann. Damit ist es beispielsweise möglich, das Verdampfen von Tröpfchen oder auch das Anwachsen von Eiskristallen zu studieren.

Aus der DE 27 09 698 A1 sind ein Verfahren und eine Vorrichtung zur Bestimmung von Dichte, Oberflächenspannung und Viskosität an kleinen Flüssigkeitsvolumina bekannt. Bei dem bekannten Verfahren, das ohne Wandkontakt durchgeführt wird, wird die zu vermessende Probe in Form eines Flüssigkeitstropfens in den Bereich einer stehenden Ultraschall-Welle geführt und dort in der Nähe eines Druckknotens der stehenden Ultraschall-Welle in der Schwebe gehalten. Die Messung der Dichte erfolgt durch ein Vergleich der Kompensationsspannungen des Ultraschall-Senderwandlers zur Positionierung der Probenflüssigkeit und einer Normierungsflüssigkeit bei exakt gleicher Position. Zur Bestimmung der Oberflächenspannung und der Viskosität wird die Probenflüssigkeit durch niederfrequente Amplitudenmodulation zu Volumenschwingungen angeregt und aus der Resonanzkurve der Volumenschwingungen werden die Oberflächenspannung und die Viskosität ermittelt. Die bekannte Vorrichtung zur Durchführung des bekannten Verfahrens besteht aus dem Ultraschall-Senderwandler und einem Reflektor zur Erzeugung der stehenden Ultraschall-Welle, einer Zugabeeinrichtung für die zu vermessende Probe bzw. für die zur Normierung benötigte Flüssigkeit, einer optischen Einrichtung zur visuellen Kontrolle der Positionierung der Probenflüssigkeit sowie einer Lichtquelle mit einem gegenüberliegend angeordneten Fotodetektor. Für den Bereich der stehenden Ultraschall-Welle kann ein heizbarer Rohrofen vorgesehen sein. Alternativ sind zur Heizung des zu vermessenden Tropfens eine Laserquelle und für die Temperaturkontrolle ein Pyrometer vorgesehen.

Aus der WO 99/44746 A1 ist ein System zum Durchführen eines Tests an einem levitierten Tropfen bekannt. Das System weist einen Levitator zum Suspendieren des Tropfens, eine Abgabeeinrichtung zum Abgeben einer Substanz an die Oberfläche des suspendierten Tropfens und ein Detektorsystem zum Detektieren von Änderungen bei dem Tropfen auf. Der Levitator ist ein akustischer Levitator.

Aus der US 2003 / 0 154 790 A1 sind ein Verfahren und eine Vorrichtung zum optischakustischen Formen bekannt. Akustische Energie wird verwendet, um die Gestalt eines Teilchens oder eines Teilchenclusters zu dem Zweck zu steuern, eine Phasen- und damit eine Dichteänderung als Resultat der Aussetzung gegenüber Strahlung zu induzieren. Ein akustische Wandler und ein akustischer Reflektor werden um das Teilchen oder den Teilchencluster herum angeordnet, um eine stehende Welle zu erzeugen. Diese stehende Welle hält das Teilchen oder den Teilchencluster in Schwebe. Dabei übt sie Kräfte auf Punkte an der Oberfläche des Teilchens aus. Der Ort solcher Punkte gibt eine dreidimensionale Druckfunktion wieder, die dazu führt, dass das Teilchen oder der Teilchencluster eine vorbestimmte Gestalt annimmt. Nachdem das Teilchen oder der Teilchencluster die gewünschte Form oder Dichte erreicht hat, induziert eine Strahlungsquelle ein schnelles Schmelzen oder Verfestigen der Teilchen. Während des Vorgangs wird das Teilchen oder der Teilchencluster mit einer Kamera beobachtet, um die Form des Teilchens oder Teilchenclusters und bei einem Teilchencluster auch die Anzahldichte des Teilchenclusters zu beobachten. Eine Erfassung der physikalischen Dichte, d. h. des Quotienten aus Masse und Volumen des Teilchens oder des Teilchenclusters wird in der US 2003 / 0 154 790 A1 nicht beschrieben.

Neben der Levitation durch Ultraschallwellen ist auch eine Levitation von Tropfen in einer aufsteigenden Strömung bekannt.

### WEITERE BESCHREIBUNG DER ERFINDUNG

Das erfindungsgemäße Verfahren wendet nicht nur die grundsätzlich bekannte Technik der Levitation beim Bestimmen der Schwindung von Harz beim Aushärten an, sondern sie kombiniert diese bekannte Technik mit einer speziellen Erfassung des Volumens des Tropfens, bei der die Form und die Abmessungen des Tropfens in allen drei Raumrichtungen erfasst werden. Das erfindungsgemäße Verfahren kommt so ohne jegliche Annahmen über eine bestimmte geometrische Form des Tropfens aus. Dadurch ist das erfindungsgemäße Verfahren gegenüber jeglichen Deformationen des Tropfens robust. Zugleich werden Einflüsse auf den Tropfen durch Kontakte mit festen Oberflächen verhindert, indem der Tropfen spätestens beginnend mit dem frühen Erfassen und mindestens bis zum Ende des späten Erfassens seines Volumens durch Levitation in Schwebe gehalten wird. Der Schwebezustand des Tropfens stellt umgekehrt sicher, dass die Form und die Abmessungen des Tropfens in allen drei Raumrichtungen zuverlässig erfassbar sind, weil der schwebende Tropfen für diese Erfassung aus allen Raumrichtungen zugänglich ist.

Bei dem erfindungsgemäßen Verfahren kann der Tropfen mit einem Volumen in einem Bereich von 1 µl und insbesondere von 5 µl bis zu 50 µl und insbesondere bis zu 10 µl ausgebildet werden. Das Volumen des Tropfens kann damit klein sein, so dass mit jeder Bestimmung der Schwindung des Harzes nur wenig von dem Harz verbraucht wird. Entsprechend geht auch das Aushärten des Harzes über das kleine Volumen hinweg schnell vonstatten. Beides ermöglicht es, mit dem erfindungsgemäßen Verfahren in begrenzter Zeit eine Vielzahl von Harzen vorgegebener Zusammensetzungen hinsichtlich ihrer Schwindung beim Aushärten zu analysieren.

Konkret können die Form und die Abmessungen des Tropfens bei dem frühen Erfassen und bei dem späten Erfassen des Volumens unter Abbilden des Tropfens in mindestens zwei verschiedenen Abbildungsrichtungen erfasst werden. Damit gleichwirkend ist es, die Form und die Abmessungen des Tropfens bei dem frühen Erfassen und bei dem späten Erfassen unter Abtasten des Tropfens mit Lichtstrahlen aus mindestens zwei verschiedenen Abtastrichtungen zu erfassen. Besonders bevorzugt ist es, die Form und die Abmessungen des Tropfens bei dem frühen Erfassen und bei dem späten Erfassen aus so viel verschiedenen Abbildungsrichtungen bzw. Abtastrichtungen zu erfassen, dass die Oberfläche des Tropfens vollumfänglich abgebildet bzw. abgetastet wird.

Die verschiedenen Abbildungsrichtungen bzw. Abtastrichtungen können durch mehrere Abbildungseinrichtungen bzw. Abtasteinrichtungen und/oder durch Rotieren des durch Levitation in Schwebe gehaltenen Tropfens realisiert werden. Das Rotieren des Tropfens kann durch die Bedingungen, unter denen der Tropfen in Schwebe gehalten wird, bewirkt werden.

Weiterhin ist es bevorzugt, wenn bei dem erfindungsgemäßen Verfahren zwischen dem frühen Erfassen und dem späten Erfassen mindestens ein weiteres Erfassen des Volumens des Tropfens während des Aushärtens des Harzes erfolgt. Darüber hinaus kann mindestens ein weiteres Erfassen des Volumens des Tropfens auch noch nach dem späten Erfassen des Volumens erfolgen, um auch eine erst später eintretende Schwindung des Harzes zu messen.

Bei dem erfindungsgemäßen Verfahren kann das Harz beim Aushärten temperiert werden. Dieses Temperieren bedeutet insbesondere ein Erwärmen des Harzes, um seine Heißhärtung auszulösen. Grundsätzlich kann aber auch gezielt eine Kalthärtung des Harzes durchgeführt und seine daraus resultierende Schwindung erfasst werden.

In jedem Fall kann das Harz beim Aushärten temperiert werden, indem ihm Energie zugeführt oder entzogen wird. das Zuführen von Energie kann konkret durch elektromagnetische Strahlung und/oder Konvektion und/oder Induktion erfolgen. Die elektromagnetische Strahlung kann dabei Laserlicht und/oder Infrarotlicht und/oder Mikrowellen umfassen. Das Entziehen von Energie kann zum Beispiel durch Abfuhr von Energie aus der Umgebung des Tropfens mit Hilfe von Kühlflächen erfolgen.

Weiterhin kann der Tropfen bei dem erfindungsgemäßen Verfahren von dem frühen Erfassen bis zu dem späten Erfassen und auch noch danach, insbesondere wenn auch noch danach ein weiteres Erfassen des Volumens des Tropfens erfolgt, in einer Atmosphäre mit vorgegebener Zusammensetzung und/oder mit vorgegebenem Druck gehalten werden. So erfolgt das Aushärten bei dem erfindungsgemäßen Verfahren unter exakt definierten Randbedingungen, und diese Randbedingungen können kontrolliert variiert werden. Mit anderen Worten schließt die Angabe, dass die Atmosphäre eine vorgegebene Zusammensetzung und/oder einen vorgegebenen Druck aufweist, nicht nur ein, dass diese Zusammensetzung und dieser Druck konstant gehalten werden, sondern auch, dass die Zusammensetzung und der Druck während des Aufheizens nach einem vorgegebenen Zeitplan variiert werden.

Neben dem Erfassen des Volumens des Tropfens kann ein Zustand des Tropfens, insbesondere während des Aushärtens, durch kontaktloses Messen mindestens einer physikalischen Größe des Tropfens erfasst werden. Bei dieser physikalischen Größe kann es sich z. B. um die Temperatur handeln, die anhand von von dem Tropfen ausgehender Wärmestrahlung erfasst wird. Zum Messen der physikalischen Größe kann aber auch ein physikalisches Testsignal eingesetzt werden, das ein physikalisches Messsignal von dem Tropfen hervorruft. Hierzu zählen Verfahren, bei denen der Tropfen zur Emission von Strahlung wie Fluoreszenzlicht oder Raman-Strahlung angeregt wird, aber auch Verfahren, bei denen die Transmission des Testsignals durch den Tropfen beobachtet wird. Dabei sind auch tomographische Messverfahren anwendbar, bei denen das Testsignal aus unterschiedlichen Richtungen auf den Tropfen gerichtet und das zugehörige Messsignal registriert wird. Mit anderen Worten kann bei dem erfindungsgemäßen Verfahren der physikalische und auch der chemische Zustand des Tropfens während des Aushärtens mit verschiedensten Techniken und damit insgesamt vollständig erfasst werden, so dass die Schwindung des Harzes einem bestimmten Schritt des Aushärtens zugeordnet werden kann.

Bei dem erfindungsgemäßen Verfahren kann der Tropfen insbesondere durch Ultraschall-Levitation aber auch durch Strömungslevitation in der Schwebe gehalten werden. Bei Anwendung von Ultraschall-Levitation kann durch den Ultraschall der Tropfen nicht nur an einem bestimmten Ort in Schwebe gehalten, sondern auch beispielsweise kugelförmig aber auch anders durch den Ultraschall geformt werden. Je nach Stärke und Ausbildung der Levitation kann der Ort des Tropfens in der Vertikalen und auch in der Horizontalen gegenüber den zum Erfassen des Volumens des Tropfens verwendeten Einrichtungen variiert werden. Somit ist eine genaue Platzierung des Tropfens in einem Erfassungsbereich dieser Einrichtungen möglich.

Bei einer erfindungsgemäßen Verwendung wird eine Vorrichtung mit einem Levitator und mit einer Kameraanordnung, die dazu ausgebildet ist, den von dem Levitator durch Levitation in Schwebe gehaltenen Tropfen aus den unterschiedlichen Abbildungsrichtungen zu erfassen, bei der Durchführung des erfindungsgemäßen Verfahrens verwendet.

Die Vorrichtung kann zusätzlich mindestens eines der folgenden Merkmale aufweisen: eine Rotationseinrichtung mit mindestens einer Rotationsachse zwischen der Kameraanordnung und dem von dem Levitator durch Levitation in Schwebe gehaltenen Tropfen und/oder mindestens zwei Kameras mit den unterschiedlichen Abbildungsrichtungen, um den von dem Levitator durch Levitation in Schwebe gehaltenen Tropfen aus den unterschiedlichen Abbildungsrichtungen zu erfassen, eine Tropfenabgabeeinrichtung, eine Heizeinrichtung, eine geschlossene Probenkammer, eine Gaszufuhreinrichtung, eine Zustandsmesseinrichtung, einen Ultraschall-Levitator, einen Strömungslevitator.

Die Rotationseinrichtung kann eine mechanische Rotationseinrichtung sein, mit der die Kameraanordnung um die mindestens eine Rotationsachse oder auch um zwei oder drei verschiedene, d. h. linear unabhängige Rotationsachsen gegenüber dem Levitator verdrehbar ist. Auch durch eine geeignete Ansteuerung des Levitators kann eine Relativdrehung bewirken, die das Erfassen des von dem Levitator durch Levitation in Schwebe gehaltenen Tropfen aus den unterschiedlichen Abbildungsrichtungen mit weniger Kameras und im Extremfall mit nur einer Kamera der Kameraanordnung ermöglicht. D. h. der Tropfen kann während des Aushärtens ruhen oder kontrolliert in mindestens einer Rotationsrichtung um die mindestens eine Rotationsachse rotiert werden, wobei die Rotationsvariante bei ausreichend hoher Drehzahl mit einer geringeren Anzahl von Kameras bzw. anderen Erfassungseinrichtungen auskommt. Die Drehzahl des Tropfens kann mit einer geeigneten Sensorik erfasst und bei der Ansteuerung des Levitators berücksichtigt werden.

Die Zustandsmesseinrichtung kann eine Lichtquelle und ein Raman-Spektrometer, ein Infrarotlichtspektrometer und auch komplexere Einrichtungen wie einen Computertomographen oder einen Magnetresonanztomographen umfassen.

Der Ultraschall-Levitator weist mindestens einen Ultraschallsender und einen Ultraschallreflektor auf. Dabei können der Ultraschallsender und der Ultraschallreflektor jeweils eine ebene oder halbschalenförmige Ultraschall-abstrahlende bzw. -reflektierende Grenzfläche aufweisen. Der Ultraschall-Levitator kann insbesondere auch mehrere Ultraschallsender und zugeordnete Ultraschallreflektoren aufweisen, die für eine Platzierung des Tropfen in der Vertikalen und der Horizontalen gezielt und getrennt voneinander ansteuerbar sind.

Der Strömungslevitator kann einen ebenen oder halbschalenförmigen Boden mit über die Fläche des Bodens verteilten Düsen aufweist, deren Verteilung und Durchströmung eine gezielte Platzierung des Tropfens erlauben, um diesen kontaktfrei zu vermessen. Die Düsen können gleiche oder unterschiedliche Durchmesser und Formen aufweisen und gleichmäßig, d. h. in gleichbleibender Anzahl pro Flächeneinheit, oder ungleichmäßig über die Fläche des Bodens verteilt sein.

Vorteilhafte Weiterbildungen der Erfindung ergeben sich aus den Patentansprüchen, der Beschreibung und den Zeichnungen. Die in der Beschreibung genannten Vorteile von Merkmalen und von Kombinationen mehrerer Merkmale sind lediglich beispielhaft und können alternativ oder kumulativ zur Wirkung kommen, ohne dass die Vorteile zwingend von erfindungsgemäßen Ausführungsformen erzielt werden müssen. Ohne dass hierdurch der Gegenstand der beigefügten Patentansprüche verändert wird, gilt hinsichtlich des Offenbarungsgehalts der ursprünglichen Anmeldungsunterlagen und des Patents Folgendes: weitere Merkmale sind den Zeichnungen - insbesondere den dargestellten Geometrien und den relativen Abmessungen mehrerer Bauteile zueinander sowie deren relativer Anordnung und Wirkverbindung - zu entnehmen. Die Kombination von Merkmalen unterschiedlicher Ausführungsformen der Erfindung oder von Merkmalen unterschiedlicher Patentansprüche ist ebenfalls abweichend von den gewählten Rückbeziehungen der Patentansprüche möglich und wird hiermit angeregt. Dies betrifft auch solche Merkmale, die in separaten Zeichnungen dargestellt sind oder bei deren Beschreibung genannt werden. Diese Merkmale können auch mit Merkmalen unterschiedlicher Patentansprüche kombiniert werden. Ebenso können in den Patentansprüchen aufgeführte Merkmale für weitere Ausführungsformen der Erfindung entfallen.

Die in den Patentansprüchen und der Beschreibung genannten Merkmale sind bezüglich ihrer Anzahl so zu verstehen, dass genau diese Anzahl oder eine größere Anzahl als die genannte Anzahl vorhanden ist, ohne dass es einer expliziten Verwendung des Adverbs "mindestens" bedarf. Wenn also beispielsweise von einem Tropfen die Rede ist, ist dies so zu verstehen, dass genau ein Tropfen, zwei Tropfen oder mehr Tropfen vorhanden sein sollen. Die in den Patentansprüchen angeführten Merkmale können durch andere Merkmale ergänzt werden oder die einzigen Merkmale sein, die das jeweilige Verfahren, die jeweilige Vorrichtung oder deren Verwendung aufweist.

Die in den Patentansprüchen enthaltenen Bezugszeichen stellen keine Beschränkung des Umfangs der durch die Patentansprüche geschützten Gegenstände dar. Sie dienen lediglich dem Zweck, die Patentansprüche leichter verständlich zu machen.

### KURZBESCHREIBUNG DER FIGUREN

Im Folgenden wird die Erfindung anhand in den Figuren dargestellter bevorzugter Ausführungsbeispiele weiter erläutert und beschrieben.
**Fig. 1** ist ein Längsschnitt durch eine Vorrichtung bei der Durchführung des erfindungsgemäßen Verfahrens.
**Fig. 2** ist ein Querschnitt durch die Vorrichtung gemäß Fig. 1.
**Fig. 3** ist ein Längsschnitt durch eine weitere Vorrichtung bei der Durchführung des erfindungsgemäßen Verfahrens.
**Fig. 4** ist ein Längsschnitt durch noch eine weitere Vorrichtung bei der Durchführung des erfindungsgemäßen Verfahrens.
**Fig. 5** zeigt einen Boden der Vorrichtung gemäß Fig. 4 in einer perspektivischen Ansicht.
**Fig. 6** ist ein Ablaufdiagramm des erfindungsgemäßen Verfahrens.

### FIGURENBESCHREIBUNG

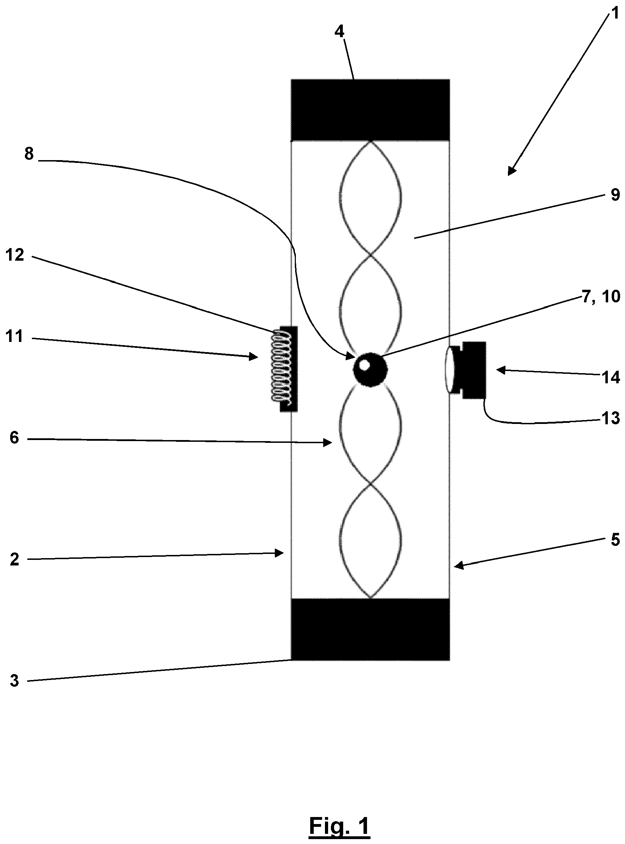

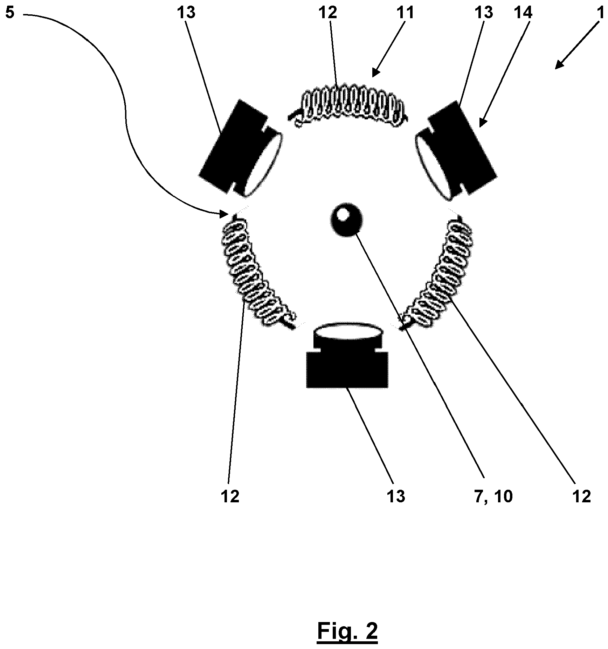

Die in den **Fig. 1** **und** **2** in einem Längsschnitt und einem Querschnitt dargestellte Vorrichtung 1 weist einen Ultraschall-Levitator 2 auf. Der Ultraschall-Levitator 2 umfasst einen Ultraschallsender 3 und einen Ultraschallreflektor 4, und er ist dazu ausgebildet, zwischen dem Ultraschallsender 3 und dem Ultraschallreflektor 4 in einem Rohrabschnitt 5 eine stehende Ultraschallwelle 6 auszubilden. Es versteht sich, dass dabei die Positionen des Ultraschallsenders 3 und des Ultraschallreflektors 4 gegenüber Fig. 1 vertauscht sein könnten. Ein Teilchen, insbesondere ein Tropfen 7, der im Bereich eines Druckknotens 8 dieser stehenden Ultraschallwelle 6 angeordnet wird, wird hier gehalten und sinkt nicht aufgrund seiner Schwerkraft in dem Rohrabschnitt 5 herab. Der Tropfen 7 behält auch in radialer Richtung des Rohrabschnitts 5 seine Position in dem Druckknoten 8 der stehenden Ultraschallwelle 6 bei. So wird der Tropfen 7 berührungslos in dem Druckknoten 8 fixiert. Der fixierte Tropfen 7 kann in dem Rohrabschnitt 5 gezielt einer bestimmten Atmosphäre 9 und einem bestimmten Druck ausgesetzt werden. Um ein Harz 10, aus dem der Tropfen 7 besteht, auszuhärten, kann der Tropfen 7 mit einer Heizeinrichtung 11 erwärmt werden. Die Heizeinrichtung 11 kann z. B. eine Infrarotlichtstrahlungsquelle, ein Mikrowellensender oder dergleichen aufweisen. Fig. 2 zeigt, dass die Heizeinrichtung 11 drei symmetrisch um die Längsachse des Rohrabschnitts 5 verteilt angeordnete Heizeinheiten 12 umfasst, wobei zwischen zwei der Heizeinheiten 12 jeweils eine Kamera 13 einer Kameraanordnung 14 angeordnet ist. Die drei Kameras 13 erfassen den Tropfen 7 aus unterschiedlichen Abbildungsrichtungen und ermöglichen so eine Erfassung von Form und Abmessungen des Tropfens in allen drei Raumrichtungen, um sein aktuelles Volumen zu erfassen. Die Erfassung des Volumens des Tropfens 7 erfolgt bei dem erfindungsgemäßen Verfahren zumindest vor und nach dem Aushärten des aus dem Harz 10 ausgebildeten Tropfens 7. Das Erfassen des Volumens kann aber auch fortlaufend während des Aushärtens erfolgen, um einen zeitlichen Verlauf der Schwindung des Harzes beim Aushärten zu bestimmen. Zusätzlich zu den Kameras 13 können auch andere Messeinrichtungen, beispielsweise Infrarotsensoren zur Bestimmung der Temperatur des Harzes 10 auf den Tropfen 7 gerichtet sein. Auch weitere physikalische Größen des Tropfens 7 bzw. des Harzes 10 können mit entsprechenden Messeinrichtungen während des Aushärtens des Harzes 10 erfasst werden.

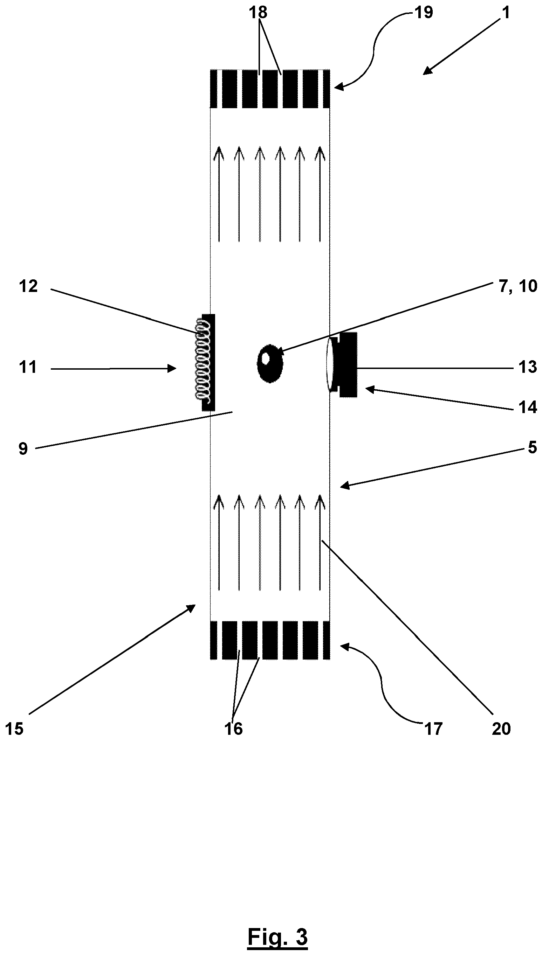

Bei der Ausführungsform der Vorrichtung 1 gemäß **Fig. 3** ist statt des Ultraschall-Levitators 2 ein Strömungslevitator 15 mit einem mit Düsen 16 versehenen Boden 17 und einem mit Öffnungen 18 versehenen Deckel 19 vorgesehen. In dem Strömungslevitator 15 wird der Tropfen 7 aus dem Harz 10 von einer nach oben gerichteten Strömung 20 in Schwebe gehalten. Dabei kann eine Temperierung des Tropfens 7 über die Temperatur dieser Strömung 20 eingestellt werden. Die Abbildung des Tropfens 7 mit den Kameras 13 der Kameraanordnung aus den verschiedenen Abbildungsrichtungen, um Form und Abmessungen des Tropfens 7 und damit sein Volumen zu erfassen, sowie das Erfassen weiterer physikalischer Größen des Tropfens 7 können in grundsätzlich gleicher Weise wie bei der Ausführungsform der Vorrichtung 1 gemäß den Fig. 1 und 2 erfolgen.

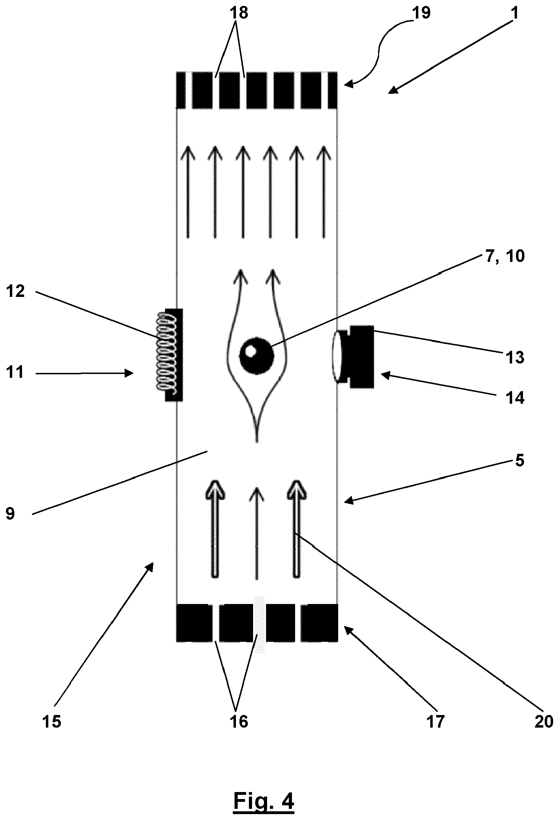

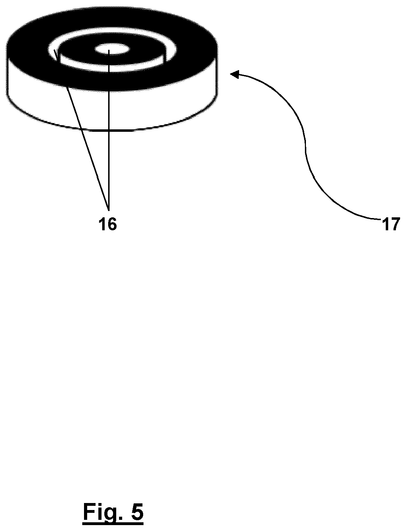

Die Ausführungsform der Vorrichtung 1 gemäß **Fig. 4** unterscheidet sich von derjenigen gemäß Fig. 3 durch eine andere Ausgestaltung des Bodens 17 mit den Düsen 16. Die Düsen 16 sind hier speziell so angeordnet, dass die nach oben gerichtete Strömung 20 auf der Achse des Rohrabschnitts 5 etwas langsamer ist als nahe der Wandung des Rohrabschnitts 5. Dadurch wird der Tropfen 7 auf der Rohrachse, d. h. auch in radialer Richtung zu dieser Rohrachse, stabilisiert. Der Boden 17 mit den Düsen 16 ist in **Fig. 5** separat in einer perspektivischen Ansicht dargestellt.

Der Boden 17 kann grundsätzlich entweder eben oder in Form einer Halbschale ausgeführt sein. Insbesondere dann, wenn der Boden 17 in Form einer Halbschale ausgeführt ist und mit punktförmigen Düsen 16 als Ausströmungslöcher versehen ist, ist es stabil möglich, den Tropfen 7 mittig in der von dem Rohrabschnitt 5 umschlossenen Probenkammer zu platzieren.

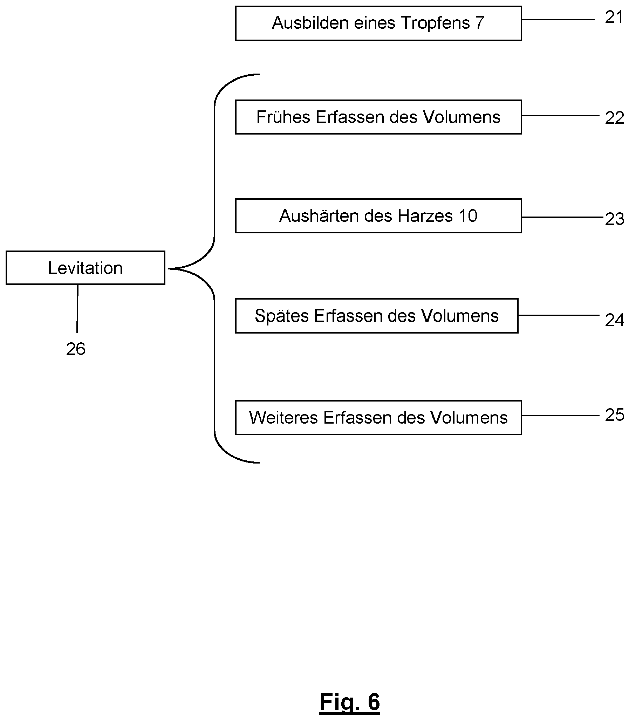

Das in **Fig. 6** anhand eines Ablaufdiagramms dargestellte Verfahren beginnt mit dem Ausbilden 21 eines Tropfens 7. Hieran schließt sich ein frühes Erfassen 22 des Volumens des Tropfens 7 an. Dann erfolgt ein Aushärten 23 des Harzes 10. Dem folgt ein spätes Erfassen 24 des Volumens. Hieran kann sich ein weiteres Erfassen 25 des Volumens anschließen. Zusätzliche Schritte des Erfassens des Volumens können während des Aushärtens 23 erfolgen. Zudem kann mindestens eine physikalische Größe des Tropfens 7 während des Aushärtens 23 fortlaufend erfasst werden. Zudem können während des Aushärtens 23 eine Umgebung des Tropfens 7 auf vorgegebene Werte eingestellt werden. Alternativ oder zusätzlich kann dem Tropfen 7 gezielt Energie zugeführt oder entzogen werden. Zumindest über die Schritte 22 bis 25 hinweg wird der Tropfen 7 durch Levitation 26 in Schwebe gehalten, d. h. berührungslos fixiert.

### BEZUGSZEICHENLISTE

- 1: Vorrichtung
- 2: Ultraschall-Levitator
- 3: Ultraschallsender
- 4: Ultraschallreflektor
- 5: Rohrabschnitt
- 6: stehende Ultraschallwelle
- 7: Tropfen
- 8: Druckknoten
- 9: Atmosphäre
- 10: Harz
- 11: Heizeinrichtung
- 12: Heizeinheit
- 13: Kamera
- 14: Kameraanordnung
- 15: Strömungslevitator
- 16: Düse
- 17: Boden
- 18: Öffnung
- 19: Deckel
- 20: Strömung
- 21: Ausbilden
- 22: frühes Erfassen
- 23: Aushärten
- 24: spätes Erfassen
- 25: weiteres Erfassen
- 26: Levitation

## Patentansprüche

1. Verfahren zum Bestimmen der Schwindung eines Harzes (10) beim Aushärten (23) mit
- Ausbilden (21) eines Tropfens (7) aus dem Harz (10),
- frühes Erfassen (22) eines Volumens des Tropfens (7) vor dem Aushärten des Harzes (10),
- Aushärten (23) des Harzes (10) und
- spätes Erfassen (24) des Volumens des Tropfens (7) nach dem Aushärten des Harzes (10)
**dadurch gekennzeichnet,**
- **dass** der Tropfen (7) von dem frühen Erfassen (22) bis zu dem späten Erfassen durch Levitation (26) in Schwebe gehalten wird und
- **dass** bei dem frühen Erfassen (22) und dem späten Erfassen (24) des Volumens Form und Abmessungen des Tropfens (7) in allen drei Raumrichtungen erfasst werden.

2. Verfahren nach Anspruch 1, **dadurch gekennzeichnet, dass** der Tropfen (7) mit einem Volumen in einem Bereich von 1 µl oder von 5 µl bis 50 µl oder bis 10 µl ausgebildet wird.

3. Verfahren nach einem der vorhergehenden Ansprüche, **dadurch gekennzeichnet, dass** die Form und die Abmessungen des Tropfens (7) bei dem frühen Erfassen (22) und bei dem späten Erfassen (24) des Volumens unter Abbilden des Tropfens (7) in mindestens zwei verschiedenen Abbildungsrichtungen und/oder unter Abtasten des Tropfens (7) mit Lichtstrahlen aus mindestens zwei verschiedenen Abtastrichtungen erfasst werden.

4. Verfahren nach einem der vorhergehenden Ansprüche, **dadurch gekennzeichnet, dass** mindestens ein weiteres Erfassen (25) des Volumens des Tropfens (7) während des Aushärtens (23) des Harzes (10) und/oder nach dem späten Erfassen (24) des Volumens erfolgt.

5. Verfahren nach einem der vorhergehenden Ansprüche, **dadurch gekennzeichnet, dass** das Harz (10) beim Aushärten (23) temperiert wird.

6. Verfahren nach Anspruch 5, **dadurch gekennzeichnet, dass** dem Harz (10) durch elektromagnetische Strahlung und/oder Konvektion und/oder Induktion Energie zugeführt wird, wobei die elektromagnetische Strahlung optional Laserlicht und/oder Infrarotlicht und/oder Mikrowellen umfasst.

7. Verfahren nach einem der vorhergehenden Ansprüche, **dadurch gekennzeichnet, dass** der Tropfen (7) von dem frühen Erfassen (22) bis zu dem späten Erfassen (24) in einer Atmosphäre (9) mit vorgegebener Zusammensetzung und/oder mit vorgegebenem Druck gehalten wird.

8. Verfahren nach einem der vorhergehenden Ansprüche, **dadurch gekennzeichnet, dass** ein Zustand des Tropfens (7) beim Aushärten (23) durch kontaktloses Messen einer physikalischen Größe des Tropfens (7) erfasst wird, wobei zum Messen der physikalischen Größe optional ein physikalisches Messsignal von dem Tropfen (7) durch ein physikalisches Testsignal hervorgerufen wird.

9. Verfahren nach einem der vorhergehenden Ansprüche, **dadurch gekennzeichnet, dass** der Tropfen (7) durch Ultraschall- oder Strömungslevitation in der Schwebe gehalten wird.

10. Verfahren nach einem der vorhergehenden Ansprüche, **dadurch gekennzeichnet, dass** der Tropfen (7) durch Ultraschall geformt wird.

11. Verwendung einer Vorrichtung (1) mit einem Levitator und mit einer Kameraanordnung (14), die dazu ausgebildet ist, den von dem Levitator durch Levitation (26) in Schwebe gehaltenen Tropfen (7) aus den unterschiedlichen Abbildungsrichtungen zu erfassen, bei der Durchführung des Verfahrens nach einem der vorhergehenden Ansprüche.

12. Verwendung nach Anspruch 11, **dadurch gekennzeichnet, dass** die Vorrichtung (1)
- eine Rotationseinrichtung mit mindestens einer Rotationsachse zwischen der Kameraanordnung (14) und dem von dem Levitator durch Levitation (26) in Schwebe gehaltenen Tropfen (7) und/oder
- mindestens zwei Kameras (13) mit den unterschiedlichen Abbildungsrichtungen und/oder
- eine Tropfenabgabeeinrichtung und/oder
- eine Heizeinrichtung (11) und/oder
- eine geschlossene Probenkammer und/oder
- eine Gaszufuhreinrichtung und/oder
- eine Zustandsmesseinrichtung und/oder
- einen Ultraschall-Levitator (2) und/oder
- einen Strömungslevitator (15)
aufweist.

13. Verwendung nach Anspruch 12, **dadurch gekennzeichnet, dass** der Ultraschall-Levitator (2) mindestens einen Ultraschallsender (3) und einen Ultraschallreflektor (4) aufweist, wobei der Ultraschallsender (3) und der Ultraschallreflektor (4) jeweils eine ebene oder halbschalenförmige Grenzfläche aufweisen.

14. Verwendung nach Anspruch 12, **dadurch gekennzeichnet, dass** der Ultraschall-Levitator (2) mehrere Ultraschallsender (3) und zugeordnete Ultraschallreflektoren (4) aufweist.

15. Verwendung nach Anspruch 12, **dadurch gekennzeichnet, dass** der Strömungslevitator (15) einen ebenen oder halbschalenförmigen Boden (16) mit über die Fläche des Bodens verteilten Düsen (17) aufweist.
